# EUROPEAN PATENT SPECIFICATION

(11) **EP 1 577 328 B1**
(45) Date of publication and mention of the grant of the patent: **11.08.2010**
(21) Application number: 03780853.2
(22) Date of filing: 17.12.2003
(51) Int. Cl.: C08F 8/30, B01J 31/06, C07C 29/40, C07C 33/30, C07C 241/02, C07C 243/38

(54) **POLYMER-IMMOBILIZED FORMAMIDES, CATALYSTS CONTAINING THE SAME AND ALLYLATION PROCESS**
AUF POLYMER IMMOBILISIERTE FORMAMIDE, KATALYSATOREN DAMIT UND ALLYLIERUNGSVERFAHREN
FORMAMIDES IMMOBILISÉS SUR UN POLYMÈRE, CATALYSEURS EN CONTENANT ET PROCÉDÉ D'ALLYLATION

(30) Priority: 17.12.2002 JP 2002365954
(43) Date of publication of application: 21.09.2005
(73) Proprietor: Japan Science and Technology Agency, Kawaguchi-shi, Saitama 332-0012 (JP)
(72) Inventor: KOBAYASHI, Shu, Chiyoda-ku, Tokyo 101-0048 (JP)
(74) Representative: Calamita, Roberto
(86) International application number: PCT/JP2003/016187
(87) International publication number: WO 2004/055066

(56) References cited:
- WO-A1-02/083606
- US-A- 3 791 996
- US-B1- 6 310 244
- KONDO SHUJI ET AL: "SYNTHESIS OF POLYMERIC FORMAMIDES AND THEIR USE AS PHASE TRANSFER CATALYSTS" J POLYM SCI POLYM LETT ED MAY 1984, vol. 22, no. 5, May 1984 (1984-05), pages 249-254, XP002397096
- KOBAYASHI, SHU ET AL: "Facile and highly stereoselective allylation of aldehydes using allyltrichlorosilanes in DMF" TETRAHEDRON LETTERS , 34(21), 3453-6 CODEN: TELEAY; ISSN: 0040-4039, 1993, XP002397097
- KOBAYASHI, SHU ET AL: "Highly Stereoselective Synthesis of Homoallylic Amines Based on Addition of Allyltrichlorosilanes to Benzoylhydrazones under Neutral Conditions" JOURNAL OF THE AMERICAN CHEMICAL SOCIETY , 121(29), 6942-6943 CODEN: JACSAT; ISSN: 0002-7863, 1999, XP002397098

## Description

### Technical Field

The present invention relates to a polymer-immobilized formamide and catalyst containing the same, and an allylation process.

### Background Art

WO 02/083606 relates to resin-conjugated polyamine combinational libraries. Kondo Shuji et al: "Synthesis of Polymeric formamides and their use as Phase Transfer Catalysts", J. Polym Sci Polym Lett, ed. May 1984, vol. 22, No. 5, May 1984 (1984-05), pages 249-254 discloses the preparation of N-methyl-N-(p-vinylbenzyl)-formamide based polymers. Kobayashi, Shu et al: "Highly Stereoselective Synthesis of Homoallylic Amines Based on Addition of Allyltrichlorosilanes to Benzoylhydrazones under Neutral Conditions", Journal of the American Chemical Society, 121(29), 6942-6943 Coden: Jacsat; ISSN: 0002-7863, 1999 describes the preparation of various homoallylic amines.

Conventionally, from the viewpoint of reducing loads on the natural environment by metal components, attention has been directed to develop and use organic catalysts which are free from metallic components. Using an organic compound as a catalyst will be a solution for the difficulty in treating metallic catalyst components in waste liquids or waste products and loads on the natural environment exerted by the metallic catalyst components.

However, in the case where an organic compound is used as a catalyst, separation of the organic catalyst from products of synthesis reaction is not easy, and separation of such organic catalyst poses a great problem in practical organic synthesis processes.

In these circumstances, immobilizing an organic compound serving as a catalytic component to a polymer so as to facilitate separation from products has been proposed. However, only few examples of polymer-immobilized organic catalysts have proved effective (Document 1).

On the other hand, the present inventors have proposed a method of synthesizing from an aldehyde compound and an allyl trihalogenosilane compound, a corresponding allyl alcohol compound by allylation in the presence of a Lewis base such as N,N-dimethylformamide (DMF) without using a metallic catalyst (Documents 2 and 3). This method is very advantageous in synthesis of an allyl alcohol compound.

With regard to this method, if the catalytic Lewis base or the like could be recovered from reaction products more efficiently, more excellent practical efficiency and catalyst recovery, as well as improved reusability would be expected.
Document 1: M. Benaglia, et al., Adv. Synth. Catal., 2002, 344, 533
Document 2: S. Kobayashi, et al., Tetrahedron Lett., 1993, 34, 3453
Document 3: S. Kobayashi, et al., J. Org. Chem. 1994, 59, 6620

In view of the foregoing circumstance, it is an object of the present invention to provide a novel organic catalyst which is useful as an organic catalyst which is free from metallic components and very easy to recover from the reaction mixture of synthesis and to reuse; a polymer-immobilized organic substance which is useful as a synthetic intermediate; a catalyst containing the same as an active ingredient; and an allylation process of aldehyde compound using the same.

In a first aspect, the present invention provides polymer-immobilized formamides represented by the following formula (1): 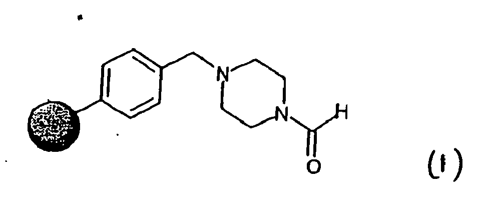 wherein the solid circle represents a cross-linked polystyrene polymer.

In a second aspect, the present invention provides a reaction catalyst comprising the foregoing polymer-immobilized formamide, as an active ingredient.

In a third aspect, the present invention provides a process for allylation of an aldehyde compound characterized by allylating an aldehyde compound with an allyl trihalogenosilane in the presence of the above polymer-immobilized formamide, thereby generating an allyl alcohol compound. The allylation may be conducted in the presence of a polar solvent.

Further, in a fourth aspect, the present invention provides a process for allylation of a hydrazone compound characterized by allylating a hydrazone compound with an allyl trihalogenosilane in the presence of the above polymer-immobilized formamide, thereby generating an allyl hydrazine compound. The allylation may be conducted in the presence of a polar solvent.

The present invention has the features as described above, and embodiments thereof will be explained below.

The solid circle shown in the general formula usually represents a cross-linked polystyrene polymer. The polymer used herein may be selected from various kinds of cross-linked polystyrene polymers that are reaction insoluble depending on the application and synthesis method, and examples of the polymer include cross-linked polystyrene polymers which are commercially available and hence easily obtainable.

The polymer-immobilized formamide of the present invention may be produced, for example, by preparing a chloromethylated polymer, and causing the same to react with formamide or its derivative. Examples of the chloromethylated polymer include those obtained by reacting cross-linked polystyrene and chloromethylmethylether in the presence of a catalytic amount of SnCl₄ (for example, S. Kobayashi, et al., Tetrahedron Lett., 1997, 38, 4251) or commercially available Merrifield' s resin. These resins may have a controlled quantity of chloromethyl groups.

In the case where a prepared chloromethylated polymer such as chloromethylated cross-linked polystyrene is used, the polymer is caused to react with N-methyl formaldehyde in DMF in the presence of NaH, to obtain a polymer-immobilized formamide with very high yield in the range of 99% to quantitative yield. Various polymer-immobilized formamides represented by the formula (1) may be produced in a similar manner.

Polymer-immobilized formamides according to the present invention may be utilized as catalysts, e.g., Lewis bases, for various organic synthetic reactions. Since they impose a small burden on the environment because they are organic catalysts free from metallic components, and they are immobilized on a polymer, they are very easy for separation, recovery from products after synthetic reaction and reuse. The polymer-immobilized formamides of the present invention are not only useful as catalysts or reaction promoters, but also applicable as intermediates of synthetic reaction.

The present invention also provides a novel method that uses the above-described polymer-immobilized formamide as a catalyst or a reaction promoter for allylation of an aldehyde compound or a hydrazone compound. To be more specific, provided is a method of synthesizing an allyl alcohol compound by causing an aldehyde compound to react with an allyl trihalogenosilane compound in the presence of the polymer-immobilized formamide of the present invention as described above. Also provided is a method for synthesizing an allyl hydrazine compound by causing a hydrazone compound to react with an allyl trihalogenosilane compound.

In the present reaction method, an aldehyde compound represented by the formula (2): 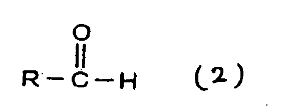 (wherein R represents an optionally substituted hydrocarbon group or heterocyclic group, and the hydrocarbon group may be chain or cyclic, or combination thereof) is caused to react, in the presence of the polymer-immobilized formamide, with an allyl trihalogenosilane compound represented by the formula (3): 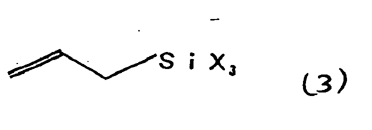 (wherein X represents a halogen atom), to synthesize an allyl alcohol compound represented by the formula (4):

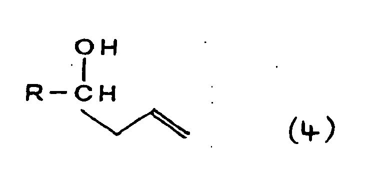

Alternatively, for example, a hydrazone compound represented by the formula (5): 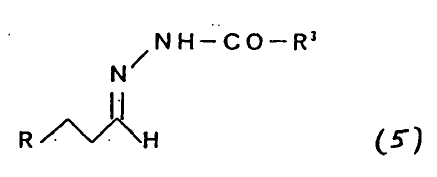 (wherein R and R³ each represents an optionally substituted hydrocarbon group or heterocyclic group, and the hydrocarbon group may be chain or cyclic, or combination thereof) is caused to react, in the presence of the polymer-immobilized formamide, with an allyl trihalogenosilane compound represented by the formula (3), to synthesize an allyl hydrazine compound represented by the formula (6):

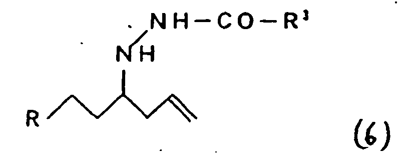

The foregoing hydrocarbon groups, heterocyclic groups, as well as substituents may be variable. Examples of such groups include alkyl groups, cycloalkyl groups, cycloalkylalkyl groups, phenyl group, phenylalkyl groups, phenyl alkenyl groups, tolyl group, naphthyl group, nitrophenyl group, methoxyphenyl group, biphenyl group, furyl group, piperidyl group and pyridinyl group.

Preferably, allylation as described above is conducted in a solvent, in particular, in a polar solvent such as nitriles, amides, sulfoxides, alcohols, halogeno hydrocarbons and the like. As a catalyst, the polymer-immobilized formamide may be generally used in an amount of more than or equal to 5% by mole, or relatively high ratio, for example about 50% by mole to 500% by mole, relative to a reaction substrate in a non-limiting manner.

Also, reaction temperature, reaction time, use ratios of aldehyde compound, hydrazone compound and allyl trihalogenosilane compound differ depending on the types thereof, and may be appropriately selected. For example, in allylation of an aldehyde compound, the reaction may be conducted at 5 to 30°C for 2 hours or more. In allylation of a hydrazone compound, it may be reacted at a temperature of equal to or less than -20°C, for example. Air or inert gas atmosphere may be used.

The present invention will be now described in more detail by way of examples. It is to be noted that the following examples will not limit the present invention.

### EXAMPLES

### <Example 1>

Cross-linked polystyrene and chloromethylmethylether were caused to react in the presence of a catalytic amount of SnCl₄, to prepare chloromethylated cross-linked polystyrene in which chloromethyl groups are introduced and bound in a range of 2.22 to 5.11 mmol/g. Commercially available Merrifield's resins included binding of chloromethyl groups of 0.63 mmol/g and 1.20 mmol/g.

These chloromethylated cross-linked polystyrenes were caused to react with N-methyl formamide under a basic condition.

The reaction was conducted in the presence of NaH in DMF solvent. Polymer-immobilized formamide (PS-Formamide 1) was obtained in yield of 99%. This product was examined for the structure by ¹³C Swollen Resin Magic Angle Spinning (SR-MAS) and IR analysis.

Likewise, polymer-immobilized formamide (PS-Formamide 2) represented by the formula (1) was obtained.

Using these products, allylation of aldehyde compound was effected according to the reaction indicated by the following formula. The results are shown in Table 1.

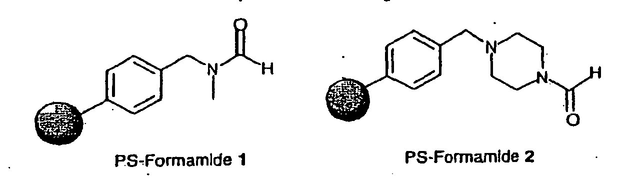

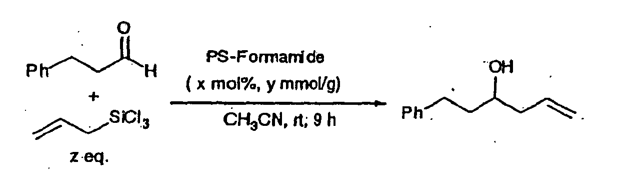

**Table 1**

| Optimization of the Reaction Conditions | | | | | | |
|---|---|---|---|---|---|---|
| Entry | Polymer | x | y | z | Conc. (M) | Yield (%). |
| 1 | 1 | 100 | 0.61 | 3 | 0.06 | 40 |
| 2 | 2 | 100 | 0.61 | 3 | 0.06 | 26 |
| 3 | 1 | 50 | 0.62 | 1.5 | 0.33 | 43 |
| 4 | 1 | 50 | 1.10 | 1.5 | 0.33 | 49 |
| 5 | 1 | 50 | 2.12 | 1.5 | 0.33 | 56 |
| 6 | 1 | 50 | 3.22 | 1.5 | 0.33 | 58 |
| 7 | 1 | 50 | 4.66 | 1.5 | 0.33 | 58 |
| 8 | 1 | 100 | 3.22 | 1.5 | 0.33 | 73 |
| 9 | 1 | 100 | 3.22 | 3 | 0.33 | 91 |
| 10 | 1 | 200 | 3.22 | 3 | 0.33 | 92 |
| 11^{a)} | 1 | 10 | 3.22 | 3 | 0.33 | 54 |
| 12 | 1 | 10 | 3.22 | 3 | 0.66 | 79 |

| | | | | | | |
|---|---|---|---|---|---|---|
| ^{a)} For 24 h. | | | | | | |

The symbols x and y represent existence ratio and existence amount of formamide derivative groups introduced and bound to cross-linked polystyrene.

The results demonstrate that PS-Formamide 1 having a ratio of more than or equal to 100% by mole give high reaction yield.

### <Example 2> (Reference Example)

Using PS-Formamide 1 having a ratio of 300% by mole of formamide derived groups as a catalyst, allylation of different kinds of aldehyde compound was conducted.

The results are shown in Table 2.

**Table 2**

| | | | | |
|---|---|---|---|---|
| 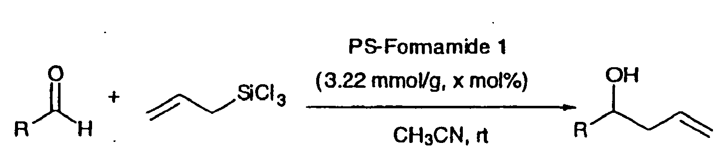 | | | | |

| Allylation of Aldehydes Using PS-Formamide 1 | | | | |
|---|---|---|---|---|
| Entry | R | x (mol%) | Time (h) | Yield (%) |
| 1 | PhCH₂CH₂- | 100 | 9 | 91 |
| 2 | Ph | 200 | 9 | 90 |
| 3 | *p*-Tol | 300 | 40 | 87 |
| 4 | *p*-NO₂Ph | 300 | 12 | 95 |
| 5 | 1-Naphtyl | 300 | 34 | 92 |
| 6 | PhCH=CH- | 300 | 12 | 66 |

### <Example 3> (Reference Example)

PS-Formamide 1 catalyst was examined for reusability, and the results are shown in Table 3.

The results of Table 3 demonstrate quantitative recovery of catalyst and high reaction yield by recycle use. The recovered PS-Formamide 1 catalyst was determined as being the same with that before use by ¹³C-NMR spectrum.

**Table 3**

| | | | |
|---|---|---|---|
| 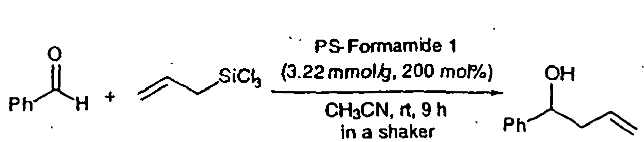 | | | |

| Reuse of PS-Formamide 1 | | | |
|---|---|---|---|
| Run | 1st | 2nd | 3rd |
| Yield (%)^{a)} | 90 (90) | 86 (85) | 86 (43) |
| Recovery (%) | quant | quant | quant |

| | | | |
|---|---|---|---|
| ^{a)} The yields obtained by using a magnetic stirrer are given in parentheses | | | |

### <Example 4> (Reference Example)

Using PS-Formamide 1 catalyst (200% by mol), allylation of hydrazone compound was conducted according to the following reaction formula, to obtain an allyl hydrazine compound in a reaction yield of 50%.

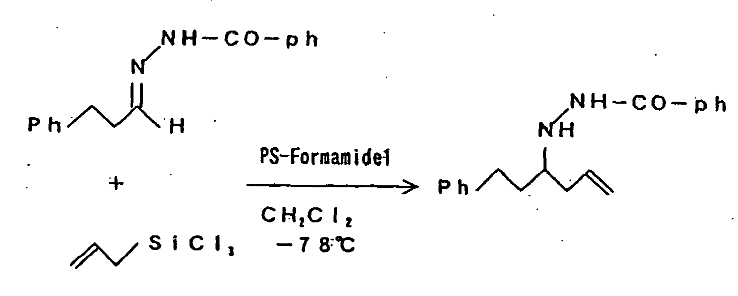

### Industrial Applicability

As specifically described above, the present invention provides a novel organic catalyst which does not use a metal component and is very easily recovered from a product of synthesis reaction and reused; a polymer-immobilized organic substance which is useful as a synthetic intermediate; a catalyst containing the polymer-immobilized organic substance as an active ingredient; a process for allylation of an aldehyde compound or a hydrazone compound in good reaction yield by using the catalyst.

## Claims

1. A polymer-immobilized formamide, **characterized by** being represented by the formula: 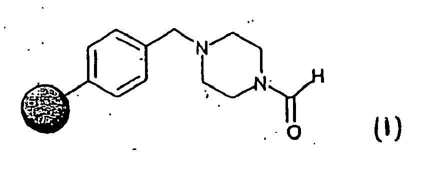 wherein the solid circle represents a cross-linked polystyrene polymer.

2. A reaction catalyst comprising the polymer-immobilized formamide according to claim 1 as an active ingredient.

3. A process for allylation of an aldehyde compound **characterized by** causing an aldehyde compound to react with an allyl trihalogenosilane in the presence of the polymer-immobilized formamide according to claim 1, to synthesize an allyl alcohol compound.

4. A process for allylation of a hydrazone compound **characterized by** causing a hydrazone compound to react with an allyl trihalogenosilane in the presence of the polymer-immobilized formamide according to claim 1, to synthesize an allyl hydrazine compound.

## Patentansprüche

1. Ein polymerimmobilisiertes Formamid, **dadurch gekennzeichnet, dass** es durch die Formel 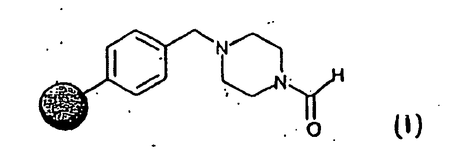 dargestellt wird, wobei der gefüllte Kreis ein vernetztes Polystyrolpolymer darstellt.

2. Ein Reaktionskatalysator, umfassend das polymerimmobilisierte Formamid gemäß Anspruch 1 als einen Wirkstoff.

3. Ein Verfahren zur Allylierung einer Aldehydverbindung, **dadurch gekennzeichnet, dass** eine Aldehydverbindung mit einem Allyltrihalogensilan in Gegenwart des polymerimmobilisierten Formamids gemäß Anspruch 1 zur Reaktion gebracht wird, um eine Allylalkoholverbindung zu synthetisieren.

4. Ein Verfahren zur Allylierung einer Hydrazonverbindung, **dadurch gekennzeichnet, dass** eine Hydrazonverbindung mit einem Allyltrihalogensilan in Gegenwart des polymerimmobilisierten Formamids gemäß Anspruch 1 zur Reaktion gebracht wird, um eine Allylhydrazinverbindung zu synthetisieren.

## Revendications

1. Formamide immobilisé sur polymère, **caractérisé en ce qu'**il est représenté par la formule 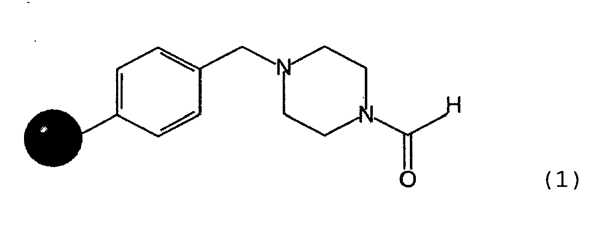 dans laquelle le cercle plein représente un polymère de polystyrène réticulé.

2. Catalyseur de réaction comprenant le formamide immobilisé sur polymère suivant la revendication 1 en tant qu'ingrédient actif.

3. Procédé d'allylation d'un composé d'aldéhyde, **caractérisé en ce que** l'on fait réagir un composé d'aldéhyde avec un allyle trihalogénosilane en présence du formamide immobilisé sur polymère suivant la revendication 1, de façon à synthétiser un composé d'alcool allylique.

4. Procédé d'allylation d'un composé d'hydrazone, **caractérisé en ce que** l'on fait réagir un composé d'hydrazone avec un allyle trihalogénosilane en présence du formamide immobilisé sur polymère suivant la revendication 1, de façon à synthétiser un composé d'allyle hydrazine.
